# EUROPÄISCHE PATENTANMELDUNG

(11) **EP 0 529 499 A1**
(43) Veröffentlichungstag der Anmeldung: **03.03.1993**
(21) Anmeldenummer: 92114145.3
(22) Anmeldetag: 19.08.1992
(51) Int. Cl.: C07K 7/18, A61K 9/08

(54) **Pharmazeutische Zusammensetzungen enthaltend Bradykinin-Antagonisten zur lokalen Anwendung an Nase und Auge**

(30) Priorität: 22.08.1991 DE 4127738
(71) Anmelder: HOECHST AKTIENGESELLSCHAFT, D-65926 Frankfurt am Main (DE)
(72) Erfinder: Seidel, Heinz-Rüdiger, Dr., W-6370 Oberursel (DE); Wirth, Klaus, Dr., W-6239 Kriftel/Taunus (DE); Giessler, Norbert, W-6000 Frankfurt am Main (DE)

(57) **Zusammenfassung**

Es werden pharmazeutische Zusammensetzungen zur lokalen Anwendung, an Nase oder Auge beschrieben, die einen Bradykinin-Antagonisten gelöst in einem physiologisch verträglichen Lösungsmittel enthalten.

## Beschreibung

Die Erfindung betrifft pharmazeutische Zusammensetzungen enthaltend Bradykinin-Antagonisten zur lokalen Anwendung an Nase und Auge, sowie Verfahren zur Herstellung solcher Zusammensetzungen.

Bradykinin (Bk) und Bradykinin-verwandte Peptide sind an vielfältigen pathologischen Zuständen des Organismus beteiligt. Sie bewirken z.B. Blutdruckabfall, Bronchokonstriktion und entzündliche Reaktionen und lösen Schmerzen aus.

Bradykinin-Antagonisten hemmen diese Wirkungen in bereits niedrigen Dosen mit einer über mehrere Stunden anhaltenden Wirkdauer. So verhindern sie einen Bk-induzierten Blutdruckabfall, heben eine Bronchokonstriktion auf und verringern Entzündungen.

Bradykinin-Antagonisten werden z.B. in EP-A-0 370 453 (entspr. US-Patentanmeldung Nr. 374 162 bzw. 746 149)beschrieben. Pharmazeutische Präparate u.a. zur nasalen Anwendung werden in allgemeiner Form beschrieben.

Es wurde nun in überraschender Weise gefunden, daß Bradykinin-Antagonisten (BkA) entzündungshemmende, schleimhautabschwellende und antiallergische Wirkungen aufweisen, wenn sie als Lösungen lokal an der Nase appliziert oder am Auge in den Bindehautsack instilliert werden. Diese Beobachtung trifft insbesondere auf den Antagonisten H-(D)-Arg-Arg-Pro-Hyp-Gly-Thi-Ser-(D)-Tic-Oic-Arg-OH (in EP-A-0 370 453 beschrieben) zu, der auch als HOE 140 bezeichnet wird. Auf diese Weise lassen sich sowohl die allergische Rhinitis als auch eine Virus-Rhinitis (normaler, banaler Schnupfen) vorteilhaft behandeln. Entzündung, Sekretion und Schwellung der Nasenschleimhäute werden reduziert. In gleicher Weise lassen sich unspezifische Konjunktivitis und allergische Konjunktivitis behandeln.

Die Aufgabe bestand nun darin, für diese Indikationen BkA enthaltende Zubereitungen zur lokalen Applikation an Nase und Auge zur Verfügung zu stellen. Weiterhin bestand die Aufgabe darin, stabile und physiologisch verträgliche Zubereitungen zu entwickeln.

Die Aufgabe wird in der Weise gelöst, daß als Kern der erfindungsgemäßen Zubereitungen BkA-Lösungen bereitgestellt werden, die entweder in Form von Tropfen oder als Dosierspray angewendet werden, oder auch in Form von Gelen und Salben.

Die Erfindung betrifft daher eine pharmazeutische Zusammensetzung zur lokalen Anwendung an Nase oder Auge, gekennzeichnet durch einen Gehalt an
a) einem Bradykinin-Antagonisten als Wirkstoff,
b) einem physiologisch verträglichen Lösungsmittel für den Wirkstoff,
c) gegebenenfalls einem physiologisch verträglichen Puffer,
d) gegebenenfalls einem Isotoniezusatz,
e) gegebenenfalls einem Konservierungsmittel,
f) gegebenenfalls einem Verdickungsmittel,
g) gegebenenfalls einer Salbengrundlage.

Unter Bradykinin-Antagonisten werden sowohl die freien Verbindungen als auch die physiologisch verträglichen Salze verstanden.

Als Bradykinin-Antagonisten kommen insbesondere die folgenden in Betracht:
1. H-D-Arg-Arg-Hyp-Pro-Gly-Thi-Ser-D-Tic-Aoc-Arg-OH
2. H-D-Arg-Arg-Pro-Hyp-Gly-Thi-Ser-D-Tic-Aoc-Arg-OH
3. H-D-Arg-Arg-Pro-Hyp-Gly-Thi-Ser-D-Tic-Tic-Arg-OH
4. H-D-Arg-Arg-Pro-Hyp-Gly-Thi-Ser-D-Tic-Oic-Arg-OH
5. H-D-Arg-Arg-Pro-Pro-Gly-Thi-Ser-D-Tic-Oic-Arg-OH
6. H-D-Arg-Arg-Pro-Hyp-Gly-Phe-Ser-D-Tic-Oic-Arg-OH
7. H-D-Arg-Arg-(NO₂)-Pro-Hyp-Gly-Phe-Ser-D-Tic-Aoc-Arg-OH
8. H-Arg(Tos)-Pro-Hyp-Gly-Thi-Ser-D-Tic-Oic-Arg-OH
9. H-D-Arg-Arg-Pro-Hyp-Gly-Leu-Ser-D-Tic-Oic-Arg-OH
10. H-D-Arg-Arg-Pro-Hyp-Gly-Thi-Ser-D-Phe-Oic-Arg-OH
Sie sind entweder in EP-A-0 370 453 beschrieben oder werden in analoger Weise wie dort in Beispiel 1 angegeben hergestellt.

Bevorzugt sind die Verbindungen 2, 4 und 8, insbesondere bevorzugt die Verbindung 4 (HOE 140).

Es ist selbstverständlich, daß die Lösungsmittel und die unter c) bis g) genannten Zusatzstoffe physiologisch verträglich sein müssen.

Bevorzugtes Lösungsmittel für die erfindungsgemäßen Zubereitungen ist Wasser. Daneben kommen aliphatische Verbindungen mit Alkoholgruppen wie Ethanol, Glyzerin oder 1,2-Propylenglykol in Frage, insbesondere auch in Gemischen mit Wasser.

Die erfindungsgemäßen Lösungen enthalten zusätzlich zum Arzneistoff zweckmäßigerweise Konservierungsmittel, Hilfsstoffe zur Einstellung der Isotonie und/oder Puffersubstanzen. Auch können Verdickungsmittel enthalten sein.

Als Konservierungsmittel sind z.B. quartäre Ammoniumverbindungen wie Cetylpyridiniumchlorid oder Benzalkoniumchlorid, Quecksilberverbindungen wie Phenylquecksilberborat, -acetat oder -nitrat oder Thiomersal-Natrium geeignet. Sie werden vorzugsweise in Konzentrationen von 0,002 - 0,05 % eingesetzt. Weitere Konservierungsmittel stellen Chlorbutanol oder Phenylethylalkohol dar, die zweckmäßigerweise in Konzentration von etwa 0,5 % allein verwendet oder mit den obengenannten Konservierungsmitteln kombiniert werden können.

Ein bevorzugtes Konservierungsmittel stellt Benzalkoniumchlorid dar. Es ist ein Alkyl-benzyl-dimethylammoniumchlorid, das ein Gemisch von Verbindungen mit Alkylkettenlängen von C8-C18 darstellt. Ergänzend zu den Konservierungsmitteln können den Lösungen noch Stabilisierungsmittel wie Ethylendiamintetraessigsäure und deren Salze (Dinatriumsalz, Calciumsalz) zugesetzt werden, die die keimabtötende Wirkung verstärken.

Zweckmäßigerweise werden zur Verbesserung der Verträglichkeit der erfindungsgemäßen Lösungen Isotonisierungsmittel zugesetzt, um den osmotischen Druck dem der physiologischen Umgebung anzugleichen. Geeignete Stoffe stellen z.B. außer Natriumchlorid mehrwertige Alkohole wie Sorbit oder Mannit dar. So sind wäßrige Lösungen von 0,9 % Kochsalz, 5,48 % Sorbit und 5,07 % Mannit isoosmotisch und weisen eine Gefrierpunktserniedrigung von 0,57°C auf. Folglich müssen die genannten Stoffe in solchen Mengen zugesetzt werden, so daß die Arzneistofflösungen diesen Wert der Gefrierpunktserniedrigung erreichen.

Ferner können die erfindungsgemäßen Zubereitungen Puffersubstanzen enthalten, mit denen der pH-Wert auf einen Bereich von 5 bis 6 eingestellt wird. Es wurde gefunden, daß sich die Puffersysteme Essigsäure/Natriumacetat und primäres/sekundäres Phosphat insbesondere eignen, um zu stabilen Lösungen zu gelangen. Die genannten Puffer werden in 0,005 - 0,05 molarer Konzentration, bevorzugt jedoch in ungefähr 0,02 molarer Menge, eingesetzt.

Überraschend war weiterhin, daß sich Lösungen mit Acetatpuffer ebenso wie die übrigen Puffer zur nasalen Applikation eignen, ohne die Nasenschleimhaut zu reizen. Dies war nicht ohne weiteres zu erwarten, wird doch Acetatpuffer im Gegensatz zum Phosphatpuffer für nasale Zubereitungen nicht ausdrücklich empfohlen (siehe H. Sucker, P. Fuchs, P. Speiser Pharmazeutische Technologie, Georg Thieme Verlag Stuttgart 1978, S. 292).

Schließlich können die Lösungen Verdickungsmittel enthalten, um ein schnelleres Abfließen zu verhindern und damit die Kontaktzeit zu verlängern. Einen derartigen Effekt bewirken z.B. Gelatine oder Natriumalginat. Bevorzugte Verdickungsmittel sind jedoch Celluloseether, Polyacrylsäure, Polyvinylalkohole sowie Polyvinylpyrrolidone. Als Celluloseether sind Methylcellulose, Hydroxypropylcellulose, Hydrorypropylmethylcellulose, Hydroryethylcellulose und Carboxymethylcellulose zu nennen. Methylcellulosen sind z.B. unter dem Warenzeichen Methocel® der Firma DOW bekannt. Die Verdickungsmittel werden in solchen Konzentrationen zugesetzt, daß die daraus hergestellten Lösungen eine leicht erhöhte Viskosität aufweisen. Beispielsweise werden von Methocel E 4M premium etwa 0,25 %, von Methocel E 15 premium etwa 1 % benötigt (Gewichtsprozent bezogen auf das Gesamtgewicht der BkA-Lösung).

Die nasale Applikation der Lösungen erfolgt durch Tropfen mit einer Pipette oder durch Versprühen aus einer Kunststoffquetschflasche. Bevorzugt ist die Anwendung in Form eines Aerosols durch Versprühen mit Hilfe einer Dosierzerstäuberpumpe.

Zur intraokularen Instillation ist die flüssige Zubereitung in gleicher Weise geeignet.

Zur weiteren Verlängerung der Verweilzeit im Auge oder in der Nase und damit der Wirkdauer ist es notwendig, noch viskosere Zubereitungen einzusetzen, wie sie Gele und Salben darstellen. So können in die schon vorstehend beschriebenen Lösungen viskositätserhöhende Stoffe (Verdickungsmittel) in solcher Konzentration eingearbeitet werden, daß gelartige, streichfähige Zubereitungen entstehen. Hierfür eignen sich z.B. die schon genannten Cellluloseether sowie Polyacrylsäure (z.B. bekannt unter dem Warenzeichen ®Carbopol). Im Falle der Celluloseether werden zweckmäßigerweise höher viskose Typen eingesetzt. So kann der Anteil an Methocel K 100 M 1 %, der an Methocel E 4 M premium 2 % betragen (Gewichts-%, bezogen auf die Lösung). Bei Verwendung der Polyacrylsäure erfolgt die Gelbildung durch Zusatz von Lauge, insbesondere von Natronlauge, bis zu einen pH von 5 - 6; sie werden in Konzentration von etwa 0,25 % (Carbopol 940) und 0,5 % (Carbopol 923) (Gewichts-% bezogen auf die Lösung) eingesetzt.

Zur Applikation am Auge sind weiterhin Salben geeignet, wobei die BkA-Lösung in die Salbengrundlage eingearbeitet wird. Als solche kommen Kohlenwassersstoffe in Frage, denen W/O-Emulgatoren zugesetzt sind. Hier sind Gemische aus flüssigem Paraffin und gelber bzw. weißer Vaseline mit Wollwachsalkoholen zu nennen. Die letzteren verleihen der Salbe die Fähigkeit, Wasser aufzunehmen und W/O-Emulsionen zu bilden. Die Wollwachsalkohole können auch durch eine Mischung aus Wollfett und Cetylalkohol ersetzt sein. Die emulgatorhaltigen Salben erlauben es, den Wirkstoff in Lösung einzuarbeiten, wobei diese auch Konservierungsstoffe enthalten, isotonisiert und gepuffert sein kann.

Die erfindungsgemäßen Zubereitungen (Lösungen, Gele, Salben) enthalten 0,0005 bis 1 % und vorzugsweise 0,001 bis 0,5 % (jeweils Gewichts-%) HOE 140, bezogen auf die freie Base bzw. einen anderen BkA in den genannten Konzentrationen.

Das Verfahren zur Herstellung der erfindungsgemäßen Zusammensetzungen ist dadurch gekennzeichnet, daß man einen Bradykinin-Antagonisten in einem physiologisch verträglichen Lösungsmittel löst und gegebenenfalls mit den unter c) bis g) genannten Hilfs- und Trägerstoffen vereinigt.

Bei Lösungen beträgt die Dosierung pro Nasenloch 0,05 bis 0,2 ml und vorzugsweise 0,1 ml, wobei die Applikation täglich mehrmals, z.B. 1 bis 5 mal täglich erfolgen kann. Die halbfesten Zubereitungen werden in einer entsprechenden Menge appliziert.

In das Auge wird üblicherweise 1 Tropfen Lösung entsprechend einem Volumen von 0,05 ml bzw. die entsprechenge Menge Gel oder Salbe gegeben.

Am Menschen lassen sich durch nasale Anwendung von Bradykinin Symptome eines Schnupfens auslösen. Besonders ausgeprägt ist die Wirkung des Bradykinins auf die Sekretion der Nase. Eine durch Bradykinin induzierte Nasensekretion konnte durch eine erfindungsgemäße Zubereitung enthaltend HOE 140 über mehrere Stunden gehemmt werden. Mit HOE 140 lassen sich deshalb sowohl die allergische Rhinitis als auch die Virusrhinitis, die durch endogen gebildete Kinine, vermittelt werden, vorteilhaft behandeln. Entzündung, Sekretion und Schwellung der Nasenschleimhaut werden reduziert. Die erfindungsgemäßen Zusammensetzungen eigenen sich daher zur Behandlung der genannten Krankheiten.

Die folgenden Beispiele sollen den Erfindungsgegenstand näher erläutern, ohne ihn jedoch einzuschränken.

### Beispiel 1

### Nasentropfen bzw. Nasenspray oder Augentropfen

| | | |
|---|---|---|
| a) | HOE 140 | 50,0 mg |
| | Essigsäure | 6,2 mg |
| | Natriumacetat x 3 H₂O | 165,5 mg |
| | Benzalkoniumchlorid | 10,0 mg |
| | Natriumchlorid | 835,0 mg |
| | Aqua bidest. ad | 100,00 g |

| | | |
|---|---|---|
| b) | HOE 140 | 50,0 mg |
| | NaH₂PO₄ x H₂O | 86,4 mg |
| | Na₂HPO₄ x 2 H₂O | 7,5 mg |
| | Benzalkoniumchlorid | 10,0 mg |
| | Natriumchlorid | 880,0 mg |
| | Aqua bidest. ad | 100,00 g |

| | | |
|---|---|---|
| c) | HOE 140 | 50,0 mg |
| | Citronensäure x H₂O | 60,0 mg |
| | Trinatriumcitrat x H₂O | 428,0 mg |
| | Benzalkoniumchlorid | 10,0 mg |
| | Natriumchlorid | 850,0 mg |
| | Aqua bidest. ad | 100,00 g |

In einem Teil Wasser werden die Puffersubstanzen, Benzalkoniumchlorid sowie Kochsalz gelöst. Der Arzneistoff wird in einem weiteren Teil Wasser gelöst. Beide Lösungen werden vereinigt und auf das gewünschte Volumen aufgefüllt. Nach dem Mischen der fertigen Lösung ergibt sich ein pH 5,5 ± 0,3. Sie wird nach Filtration über ein Membranfilter der Porenweite 0,22 µm in Glasbehälter abgefüllt, die mit einer Dosierzerstäuberpumpe verschlossen werden. Beim Betätigen der Pumpe werden 100 µl Spray entsprechend 50 µg HOE 140 in die Nase ausgebracht.

Versieht man die gefüllten Glasbehälter mit einer Pipette, kann die Lösung in die Nase oder das Auge getropft werden.

Die genannten Lösungen wurden einer Stabilitätsprüfung unterzogen. Bei einer Lagertemperatur von 40°C wurden nach 3 (bzw. 6) Monaten folgende Gehaltswerte für HOE 140 gefunden : a) 99,5 % (95,7 %); b) 98,7 % (92,9 %); c) 96,4 % (89,9 %). Die Werte zeigen die besondere Eignung der Acetat- bzw. Phosphatpuffer enthaltenden Lösungen.

### Beispiel 2

### Gel zur Anwendung an Nase bzw. Auge

| | | |
|---|---|---|
| a) | HOE 140 | 50,00 mg |
| | Essigsäure | 6,24 mg |
| | Natriumacetat x 3 H₂O | 165,50 mg |
| | Benzalkoniumchlorid | 10,00 mg |
| | Natriumchlorid | 835,00 mg |
| | Methocel E 4 M | 1500,00 mg |
| | Aqua bidest. ad | 100,00 g |

Methocel E 4 M wird in ca. 60 g Wasser dispergiert und autoklaviert. Es entsteht ein Gel, zu dem eine durch ein Membranfilter der Porenweite 0,22 µm filtriete wäßrige Lösung der Puffersubstanzen, Benzalkoniumchlorid, Kochsalz sowie HOE 140 gegeben wird. Nach Zugabe des restlichen Wassers wird bis zur Homogenität gerührt. Das transparente Gel wird in Tuben mit verlängerter Applikationsspitze abgefüllt.

| | | |
|---|---|---|
| b) | HOE 140 | 50,00 mg |
| | Benzalkoniumchlorid | 10,00 mg |
| | Sorbit | 5500,00 mg |
| | Carbopol 940 | 250,00 mg |
| | Natriumhydroxid | q.s. |
| | Aqua bidest. ad | 100,00 g |

Carbopol 940 wird in einer Lösung des Benzalkoniumchlorid und Sorbit in ca. 50 g Wasser dispergiert und autoklaviert. Nach Abkühlen der Dispersion wird eine durch ein Membranfilter der Porenweite 0,22 µm filtrierte Lösung von HOE 140 in ca. 40 g Wasser zugegeben.

Unter Rühren wird 2,5 n Natronlauge bis zu einem pH von 5,5 zugesetzt, wobei Gelbildung erfolgt. Mit Wasser wird bis zum Endgewicht aufgefüllt. Das Gel wird bis zur Homogenität gerührt und in kleine Tuben mit ausgezogener Applikationsspitze abgefüllt.

### Beispiel 3

### Salbe zur Anwendung an Auge oder Nase

| | |
|---|---|
| HOE 140 | 50,00 mg |
| Benzalkoniumchlorid | 10,00 mg |
| Aqua bidest. | 10,00 g |
| Paraffin dickflüssig | 27,0 g |
| Vaseline, weiß | 54,2 g |
| Wollfett | 6,3 g |
| Cetylalkohol | 2,5 g |

Die vier Bestandteile der Salbengrundlage werden nach Hitzesterilisation auf dem Wasserbad geschmolzen und bis zum Erkalten gerührt. In die Salbengrundlage wird eine durch ein Membranfilter der Porenweite 0,22 µm filtrierte Lösung von HOE 140 und Benzalkoniumchlorid in Wasser portionsweise eingetragen und emulgiert. Die weiche Salbe wird in kleine Tuben, die zur Anwendung an der Nase eine verlängerte Applikationsspitze aufweisen können, abgefüllt.

## Patentansprüche

1. Pharmazeutische Zusammensetzung zur lokalen Anwendung an Nase oder Auge, gekennzeichnet durch einen Gehalt an
a) einem Bradykinin-Antagonisten als Wirkstoff,
b) einem physiologisch verträglichen Lösungsmittel für den Wirkstoff,
c) gegebenenfalls einem physiologisch verträglichen Puffer,
d) gegebenenfalls einem Isotoniezusatz,
e) gegebenenfalls einem Konservierungsmittel,
f) gegebenenfalls einem Verdickungsmittel,
g) gegebenenfalls einer Salbengrundlage.

2. Zusammensetzung gemäß Anspruch 1, dadurch gekennzeichnet, daß sie als Bradykinin-Antagonisten
H-D-Arg-Arg-Hyp-Pro-Gly-Thi-Ser-D-Tic-Aoc-Arg-OH,
H-D-Arg-Arg-Pro-Hyp-Gly-Thi-Ser-D-Tic-Aoc-Arg-OH,
H-D-Arg-Arg-Pro-Hyp-Gly-Thi-Ser-D-Tic-Tic-Arg-OH,
H-D-Arg-Arg-Pro-Hyp-Gly-Thi-Ser-D-Tic-Oic-Arg-OH,
H-D-Arg-Arg-Pro-Pro-Gly-Thi-Ser-D-Tic-Oic-Arg-OH,
H-D-Arg-Arg-Pro-Hyp-Gly-Phe-Ser-D-Tic-Oic-Arg-OH,
H-D-Arg-Arg-(NO₂)-Pro-Hyp-Gly-Phe-Ser-D-Tic-Aoc-Arg-OH,
H-Arg(Tos)-Pro-Hyp-Gly-Thi-Ser-D-Tic-Oic-Arg-OH,
H-D-Arg-Arg-Pro-Hyp-Gly-Leu-Ser-D-Tic-Oic-Arg-OH oder
H-D-Arg-Arg-Pro-Hyp-Gly-Thi-Ser-D-Phe-Oic-Arg-OH enthält.

3. Zusammensetzung gemäß Anspruch 1, dadurch gekennzeichnet, daß sie als Bradykinin-Antagonisten H-D-Arg-Arg-Pro-Hyp-Gly-Thi-Ser-D-Tic-Oic-Arg-OH (HOE 140) enthält.

4. Zusammensetzung gemäß Anspruch 1, dadurch gekennzeichnet, daß der Bradykinin-Antagonist HOE 140 ist, das Lösungsmittel Wasser ist und der physiologisch verträgliche Puffer Natriumacetat ist.

5. Verfahren zur Herstellung einer Zusammensetzung gemäß Anspruch 1, dadurch gekennzeichnet, daß man einen Bradykinin-Antagonisten in einem physiologisch verträglichen Lösungsmittel löst und gegebenenfalls mit einem physiologisch verträglichen Puffer, einem Isotoniezusatz, einem Konservierungsmittel, einem Verdickungsmittel und/oder einer Salbengrundlage vereinigt.

## Patentansprüche (Patentansprüche für folgende(n) Vertragsstaat(en): ES, GR)

1. Verfahren zur Herstellung einer pharmazeutischen Zusammensetzung zur lokalen Anwendung an Nase oder Auge, dadurch gekennzeichnet, daß man einen Bradykinin-Antagonisten in einem physiologisch verträglichen Lösungsmittel löst und gegebenenfalls mit einem physiologisch verträglichen Puffer, einem Isotoniezusatz, einem Konservierungsmittel, einem Verdickungsmittel und/oder einer Salbengrundlage vereinigt.

2. Verfahren gemäß Anspruch 1, dadurch gekennzeichnet, daß man als Bradykinin-Antagonisten
H-D-Arg-Arg-Hyp-Pro-Gly-Thi-Ser-D-Tic-Aoc-Arg-OH,
H-D-Arg-Arg-Pro-Hyp-Gly-Thi-Ser-D-Tic-Aoc-Arg-OH,
H-D-Arg-Arg-Pro-Hyp-Gly-Thi-Ser-D-Tic-Tic-Arg-OH,
H-D-Arg-Arg-Pro-Hyp-Gly-Thi-Ser-D-Tic-Oic-Arg-OH,
H-D-Arg-Arg-Pro-Pro-Gly-Thi-Ser-D-Tic-Oic-Arg-OH,
H-D-Arg-Arg-Pro-Hyp-Gly-Phe-Ser-D-Tic-Oic-Arg-OH,
H-D-Arg-Arg-(NO₂)-Pro-Hyp-Gly-Phe-Ser-D-Tic-Aoc-Arg-OH,
H-Arg(Tos)-Pro-Hyp-Gly-Thi-Ser-D-Tic-Oic-Arg-OH,
H-D-Arg-Arg-Pro-Hyp-Gly-Leu-Ser-D-Tic-Oic-Arg-OH oder
H-D-Arg-Arg-Pro-Hyp-Gly-Thi-Ser-D-Phe-Oic-Arg-OH verwendet.

3. Verfahren gemäß Anspruch 1, dadurch gekennzeichnet, daß man als Bradykinin-Antagonisten H-D-Arg-Arg-Pro-Hyp-Gly-Thi-Ser-D-Tic-Oic-Arg-OH (HOE 140) verwendet.

4. Verfahren gemäß Anspruch 1, dadurch gekennzeichnet, daß man als Bradykinin-Antagonisten HOE 140, als Lösungsmittel Wasser und als physiologisch verträglichen Puffer Natriumacetat einsetzt.
